(19) Europäisches Patentamt
European Patent Office
Office européen des brevets

(11) Veröffentlichungsnummer: **0 267 877 A2**

(12) **EUROPÄISCHE PATENTANMELDUNG**

(21) Anmeldenummer: **87810645.9**

(22) Anmeldetag: **09.11.87**

(51) Int. Cl.[4]: **C 09 B 67/52**
C 09 B 67/10
// C09B48/00

(30) Priorität: **13.11.86 US 929867**

(43) Veröffentlichungstag der Anmeldung:
**18.05.88 Patentblatt 88/20**

(84) Benannte Vertragsstaaten:
**BE DE ES FR GB IT NL**

(71) Anmelder: **CIBA-GEIGY AG**
**Klybeckstrasse 141**
**CH-4002 Basel (CH)**

(72) Erfinder: **Jaffe, Edward Ephraim, Dr.**
**3 Crenshaw Drive**
**Wilmington Delaware 19810 (US)**

(54) **Verfahren zur Herstellung einer gelbstichigroten Modifikation von gamma-Chinacridon.**

(57) Verfahren zur Umwandlung von rohem Chinacridon der $\gamma_{II}$-Modifikation in Chinacridon der $\gamma_{I}$-Modifikation mit einer durchschnittlichen mittleren Partikelgröss von mehr als 0,1 µm und einer spezifischen Oberfläche von weniger als 30 $m^2/g$, dadurch, dass man entweder

a) das rohe $\gamma_{II}$-Chinacridon in Gegenwart einer zur Umwandlung genügenden Menge eines Alkohols und einer Base mahlt oder

b) das rohe $\gamma_{II}$-Chinacridon einer Vorvermahlung in die rohe $\gamma_{I}$-Modifikation unterwirft und das vermahlene Produkt mit einer zur Wachstumsförderung der Partikel genügenden Menge eines Alkohols und einer Base behandelt und anschliessend das gelbstichigrote $\gamma_{I}$-Chinacridon isoliert.

Das wünschenswerte gelbstichigrote Chinacridon der $\gamma_{I}$-Modifikation kann durch das neue Verfahren statt in mehreren Stufen in eine oder zwei Stufen und ohne Verwendung schwer zu handhabender Chemikalien, wie z.B. konzentrierte Mineralsäuren und N-Methylpyrrolidon, erhalten werden.

EP 0 267 877 A2

**Beschreibung**

Verfahren zur Herstellung einer gelbstichigroten Modifikation von $\gamma$-Chinacridon

Die vorliegende Anmeldung betrifft eine neue Verfahrensweise zur Herstellung von gelbstichigrotem $\gamma_{I}$-Chinacridon.

Vom linear-trans-Chinacridon 7,14-Dioxo-5,7,12,14-tetrahydro-chinolino-[2,3-b]-acridin der Formel

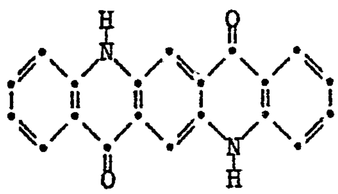

sind drei polymorphe Modifikationen bekannt. Die $\alpha$-Modifikation, wie sie in der US-PS 2 844 484 beschrieben ist und die $\gamma$-Modifikation, die in den US-PS 2 844 581 und 2 969 366 beschrieben ist, sind blaustichigrote Pigmente, während die $\beta$-Modifikation, wie sie in der US-PS 2 844 485 beschrieben ist, ein violettes Pigment darstellt. Alle sind bekannt für ihre hohe Unlöslichkeit und insbesondere für ihre ausgezeichnete Wetterechtheit. Vor einiger Zeit wurde erkannt, dass $\gamma$-Chinacridon seinerseits in zwei Modifikationen vorkommen kann (vgl. US-PS 3 074 950 und DE-AS 1 177 286), eine ältere, blaustichigrote Modifikation, wie sie in der US-PS 2 844 581 beschrieben ist und eine in jüngerer Zeit erkannte gelbstichigrote Modifikation, wie sie in der US-PS 3 074 950 offenbart wird.

Diese Farbunterschiede sind bezeichnend für Pigmente von relativ grosser Teilchengrösse, d.h. solche die eine durchschnittliche mittlere Partikelgrösse über 0,1 µm besitzen, bei der ein Maximum an Deckkraft und Wetterechtheit erreicht wird. Die zwei Modifikationen unterscheiden sich durch ihr Röntgenbeugungsdiagramm. Die "$\gamma_{II}$" genannte, blaustichigrote Modifikation zeigt drei kräftige Linien bei einem doppelton Glanzwinkel ($2\theta$) von 6,6°, 13,9° und 26,3°; fünf mittlere Linien bei 13,2°, 13,4°, 23,6°, 25,2° und 28,3° und zwei schwache Linien bei 17,1° und 20,4° $2\theta$. Die gelbere und farbstärkere, "$\gamma_{I}$" genannte Modifikation zeigt drei kräftige Linien bei einem doppelten Glanzwinkel von 6,6°, 13,9° und 26,5°, drei mittlere Linien bei 13,2°, 13,5° und 23,8° und vier schwache Linien bei 17,1°, 20,5°, 25,2° und 28,6° $2\theta$. Der bedeutendste, am leichtesten erkennbare Unterschied liegt im ausgeprägten Triplett zwischen 13° und 14° $2\theta$. $\gamma_{II}$ zeigt zwei durch 0,2 $2\theta$ getrennte Linien von annähernd gleicher Intensität bei 13,2° und 13,4° $2\theta$, wobei beide eine geringere Intensität aufweisen, als die Linie bei 13,9°. $\gamma_{I}$ andererseits, zeigt einen etwas weiteren Abstand zwischen den zwei $2\theta$ Linien bei 13,2° und 13,5°, wobei alle drei Linien bei zunehmendem doppelten Glanzwinkel an Intensität zunehmen. Die $\gamma_{I}$-Modifikation zeichnet sich zudem durch bessere Deckkraft, gute Allgemeinechtheiten, ausgezeichnete Licht- und Wetterechtheit, Hitzebeständigkeit und Beständigkeit gegen den Einfluss von Lösungsmitteln und Weichmachern aus.

Die in der US-PS 3 074 950 beschriebenen Herstellungsverfahren für die neue $\gamma$-Modifikation des Chinacridons umfassen im allgemeinen mehrere Stufen, wie Auflösen in Schwefelsäure, Verdünnen mit Wasser oder Alkohol, Salzmahlung und Behandlung mit N-Methylpyrrolidon oder Salzmahlung in Gegenwart des Pyrrolidons und eines wasserlöslichen anorganischen Salzes oder Vermischen des Chinacridon-Filterkuchens mit dem Pyrrolidon und anschliessendes Heizen und Destillieren. Die Nachteile dieser Methoden bestehen in der Notwendigkeit in mehreren Stufen zu arbeiten, in der Verwendung konzentrierter Schwefelsäure und eines hochsiedenden Pyrrolidons und in den sich daraus ergebenden ungünstigen wirtschaftlichen Auswirkungen.

Das in der DE-AS 1 177 268 beschriebene Verfahren geht von dem für die Herstellung von Chinacridon erforderlichen Zwischenprodukt aus. So wird 2,5-Dianilinoterephthalsäure mit Benzoylchlorid als ringschliessendes Mittel und Nitrobenzol als Lösungsmittel, in Gegenwart von N-Methylpyrrolidon (zur Phasenkontrolle) versetzt. Auch in diesem Falle werden schwer zu handhabende Chemikalien verwendet und die Einstellung der Korngrössenverteilung ist mit Schwierigkeiten verbunden.

Es ist nun gefunden worden, dass man $\gamma_{II}$-Chinacridon überraschenderweise, sei es in einem Einstufenverfahren, durch Mahlen in einem Alkohol in Gegenwart einer Base, oder in einem Zweistufenverfahren durch Vorvermahlen und anschliessender Behandlung mit einem Alkohol in Gegenwart einer Base, durch Kochen am Rückfluss oder durch Mahlen, in $\gamma_{I}$-Chinacridon mit einer durchschnittlichen mittleren Partikelgrösse von mehr als 0,1 µm und einer spezifischen Oberfläche von weniger als 30 $m^2/g$, ohne Einbusse der Eigenschaften umwandeln kann.

Man erhält in dieser Weise ein hochkristallines, farbstarkes, gelbstichigrotes gut dispergierbares $\gamma_{I}$-Pigment hoher Deckkraft. Beide Verfahrensvarianten führen zu Produkten mit den erwünschten Eigenschaften.

Eine erste Ausführungsform der vorliegenden Erfindung umfasst danach ein Verfahren zur Umwandlung von rohem Chinacridon der $\gamma_{II}$-Modifikation in Chinacridon der $\gamma_{I}$-Modifikation mit einer durchschnittlichen mittleren Partikelgrösse von mehr als 0,1 µm und einer spezifischen Oberfläche von weniger als 30 $m^2/g$, dadurch gekennzeichnet, das man das rohe $\gamma_{II}$-Chinacridon in Gegenwart einer zur Umwandlung genügenden Menge eines Alkohols und einer Base mahlt und anschliessend das gelbstichigrote $\gamma_{I}$-Chinacridon isoliert.

Die zweite Ausführungsform der vorliegenden Anmeldung betrifft ein Verfahren zur Umwandlung von rohem Chinacridon der $\gamma_{II}$-Modifikation in Chinacridon der $\gamma_{I}$-Modifikation mit einer durchschnittlichen mittelren Partikelgrösse von mehr als 0,1 µm

und einer spezifischen Oberfläche von weniger als 30 $m^2/g$, dadurch gekennzeichnet, dass man

a) das rohe $\gamma_{II}$-Chinacridon einer Vorvermahlung zur Umwandlung in die rohe $\gamma_I$-Modifikation unterwirft und

b) das vorvermahlene Produkt mit einer zur Wachstumsförderung der Partikel genügenden Menge eines Alkohols und einer Base behandelt und anschliessend das gelbstichigrote $\gamma_I$-Chinacridon isoliert.

Das einstufige Verfahren wird im allgemeinen z.B. wie folgt durchgeführt: Das rohe $\gamma_{II}$-Chinacridon, Alkohol und Base werden in einer geeigneten Mühle mit üblichen Mahlelementen bei einer Temperatur zwischen 20° und 50°C, bevorzugt bei Raumtemperatur, während 24-96 Stunden gemahlen. Anschliessend wird das erhaltene $\gamma_I$-Chinacridon nach üblichen Methoden isoliert.

Geeignete Alkohole sind beispielsweise niedrigsiedende Alkanole, wie Methanol, Ethanol, n-Butanol und n-Pentanol und ferner Glykole, wie Ethylenglykol. Bevorzugt wird Methanol.

Geeignete Basen sind z.B. Alkalimetallhydroxide, wie Kaliumhydroxid, Natriumhydroxid und Lithiumhydroxid, wobei Kaliumhydroxid bevorzugt wird, da es das Wachstum der Partikel und die Farbstärke des Pigments optimal fördert.

Der Alkohol ist zweckmässig in einer 6,6-bis 18-fachen, bevorzugt 10- bis 16-fachen und insbesondere 16-fachen Menge, bezogen auf das Gewicht des Pigments vorhanden. Die Base wird zweckmässig als wässrige Lösung in einer Menge von 1,0 bis 10,0 Gew-%, bevorzugt 2,2-4,5 Gew-% und insbesondere 3,3 Gew-% bezogen auf das Alkohol, eingesetzt.

Im zweistufigen Verfahren betrifft die erste Stufe das Vorvermahlen des rohen $\gamma_{II}$-Chinacridons zur Umwandlung in die $\gamma_I$-Modifikation, bei gleichzeitiger Verkleinerung der Partikelgrösse. Das erwünschte Wachstum der Partikel erfolgt in der zweiten Stufe, wobei dies mit einem grösseren Ansatz an Pigment und in kürzerer Zeit, als im einstufigen Verfahren geschehen kann. Das fehlende Vormahlen im einstufigen Verfahren hat demnach ein Arbeiten mit kleineren Pigmentmengen über etwas längere Zeit zur Folge, um Produkte mit den gleichen Eigenschaften, wie die nach dem zweistufigen Verfahren hergestellten zu erhalten.

Unter Vorvermahlen, gemäss der vorliegenden Erfindung, versteht man ein Mahlen unter Ausschluss von Flüssigkeiten oder, wenn Flüssigkeiten, wie z.B. phasenlenkende Lösungsmittel oder oberflächenaktive Mittel, verwendet werden, dann nur in so kleinen Mengen (höchstens 10,0 Gew-%, bezogen auf das Pigment) oder von solcher Beschaffenheit, dass das Pigment beim Mahlen als Pulver vorliegt.

Verschiedene Vorvermahlmethoden sind dem Fachmann geläufig. Es ist z.B. üblich in Gegenwart von Stahlkugeln (Ø 12,7 mm), von Nägeln oder, um ein Metallabrieb und die daraus folgende Notwendigkeit einer Extraktion mit verdünnter Säure zu vermeiden, von Keramikkugeln oder -stäben (Diamonite Products Manufacturing, Inc.) zu mahlen. Die Vorvermahlung kann auch mit den gleichen Mahlkugeln, die für die zweite Stufe verwendet werden, durchgeführt werden. Mahlkügelchen von 1,6 bis 2,5 mm Durchmesser, die aus einer kristallinen Zirkonium- und einer amorphen Kieselsäurephase der entsprechenden Oxide hergestellt werden, sind besonders bevorzugt (Produkte der Quartz Products Corporation). Obwohl Mahlkugeln verschiedener Grössen verwendet werden können, eignen sich die Kügelchen des obenerwähnten Grössenbereichs sehr gut für die Mahlprozesse beider Stufen dieses Verfahrens. Dieselben Mahlhilfsmittel sind auch für das einstufige Verfahren geeignet.

Die $\gamma_I$-Modifikation kann wohl durch Vorvermahlen mit Stahlkugeln von 12,7 mm oder mit Stahlschrot von 3,2 mm erhalten werden, aber die Farbstärke und der Gelbstich werden dabei wegen des Metallabriebs und der daraus folgenden Notwendigkeit einer Extraktion mit verdünnter Säure etwas geschwächt. Metallspäne oder -pulver, die während des Mahlens mit metallischen Mahlhilfsmitteln gebildet werden, müssen entfernt werden. Dies wird gewöhnlich durch Ansäuern der am Ende der Behandlung anfallenden Pigmentsuspension zu einem pH von 1 bis 2 mit einer Mineralsäure und anschliessendem Aufschluss bei 80-90°C zur vollständigen Entfernung des Metalls bewerkstelligt. Beim Kontakt von $\gamma$-Chinacridon mit heisser verdünnter Säure entsteht eine merkliche Nuancenverschiebung in Richtung blaustichigrot. Darum werden im zweistufigen Verfahren, zur Vermeidung der Extraktion mit Säure, sowohl das Vorvermahlen, wie auch der Mahlvorgang in der alkoholisch-basischen Phase, mit nichtmetallischen Mahlhilfsmitteln vorgezogen.

Wie die Veränderung des Röntgenbeugungsdiagramms zeigt, wird während der Trockenmahlung die $\gamma$-II-Modifikation in die $\gamma_I$-Modifikation umgewandelt. Die Umwandlung vollzieht sich schnell, im allgemeinen innert 12 Stunden relativ schonenden Mahlens. Das erhaltene Produkt zeigt eine bedeutend kleinere Partikelgrösse und eine geringere Kristallinität als das Ausgangsprodukt. Im Vollton ist das vorvermahlene Pigment dunkler, stumpfer und transparenter als das Ausgangsprodukt der $\gamma_{II}$-Modifikation. Die Erfahrung hat gezeigt, dass die $\gamma_I$-Form weniger hitzestabil ist als die $\gamma_{II}$-Form. Bei verlängerter Mahldauer oder bein Einsetzen von etwa 1/4 der üblichen Pigmentmenge wird das Pigment in die $\alpha$-Modifikation umgewandelt, die am wenigsten thermostabile Form aller Chinacridone. Wenn sie einmal gebildet ist, ist die $\gamma_I$-Phase die stabilste und bevorzugte Phase, sowohl im alkoholisch-basischen Medium der Flüssigmahlungsstufe als auch nach Einarbeitung des Pigments in Dispersionen für die Herstellung von Auto- und Industrielacke.

In der zweiten Stufe des zweistufigen Verfahrens wird das in der ersten Stufe hergestellte $\gamma_I$-Chinacridon von relativ niedriger Kristallinität, durch eine für die Partikel wachstumsfördernde Behandlung mit einem eine Base enthaltenden Alkohol in einem farbstarken gelbstichigroten Pigment hoher Kristallinität und relativ hoher Deckkraft umgewandelt. Dies geschieht entweder durch Mahlen mit oder durch Kochen am Rückfluss im Alkohol/Base-Gemisch.

Durch Kochen am Rückfluss im Alkohol/Base-Gemisch erhält man ein etwas weniger brillantes Produkt als durch Mahlen im gleichen Medium. Geeignete Alkohole sind beispielsweise, wie oben bereits für das einstufige Verfahren angegeben, niedrigsiedende Alkanole, wie Methanol, Ethanol, Butanol und Pentanol und ferner Glykole, wie Ethylenglykol. Bevorzugt ist Methanol. Geeignete Basen sind ebenfalls wie für das einstufige Verfahren z.B. Alkalimetallhydroxide, wie Natriumhydroxid, Lithiumhydroxid und insbesondere Kaliumhydroxid.

Die Konzentrationen an Base und Alkohol sind die gleichen wie oben für das einstufige Verfahren angegeben. Sie sind so ausgewählt, dass man damit ein Optimum an Farbstärke im Vollton erreicht. Bei der angegebenen Basenkonzentration ist keine Bildung von Kaliumsalz des Chinacridons ersichtlich, das wegen seiner deutlich blauen Farbe im Vergleich zur roten Farbe des Pigments leicht erkennbar wäre. Der Temperaturbereich für die Nassvermahlung-Stufe liegt zwischen 20° und 50°C, bevorzugt zwischen 20° und 30°C. Je höher die Temperatur beim Vermahlen, umso grösser die erhaltenen Pigmentpartikel.

Das Vermahlen des trockenen Pigments kann in Gegenwart verschiedener Zusatzstoffe, z.B. anorganische Salze, wie wasserfreies Natriumsulfat, durchgeführt werden. Durch die Anwesenheit des Natriumsulfat wird beispielsweise die Explosionstendenz des erzeugten Mahlpulvers unterbunden. Bei Verwendung von Stahl- oder Keramikkügelchen des oben als bevorzugt angegebenen Grössenbereichs ist allerdings der Zusatz von Natriumsulfat nicht wesentlich, da das Mahlpulver ziemlich zusammengeballt und nicht staubig vorliegt. Auch die Isolierung des Pigmentpulvers wird durch den Einsatz solcher Mahlhilfsmittel, sei es beim Trocken- wie beim Nassmahlen, erleichtert.

Wenn erwünscht können für den Alkohol/Base-Mahlvorgang (sowohl im ein- wie im zweistufigen Verfahren) oberflächenaktive Mittel, Verdünnungsmittel oder wachstumshemmende Mittel zugesetzt werden, unter der Bedingung, dass diese Additive durch das basische Medium nicht desaktiviert werden. Der einfache Zusatz von anionischen, kationischen oder nichtionogenen Tensiden kann z.B. die Deckkraft der erfindungsgemäss hergestellten Pigmente noch zusätzlich verbessern.

Obschon die endgültige Partikelgrösse ($>$ 0,1 μm; bevorzugt 0,2-0,7 μm während des Nassmahlvorganges erzeugt wird und deshalb das Produkt direkt aus der Mahlaufschlämmung nach Entfernung der Mahlhilfsmittel isoliert werden könnte, wird das Pigment am besten dadurch isoliert, dass man die Pigmentaufschlämmung von den Mahlhilfsmitteln durch Verdünnung und Auswaschen mit Alkohol und/oder Wasser befreit und anschliessend den Alkohol abdestilliert. Der Alkohol kann somit zurückgewonnen werden und das Pigment durch Filtrieren der zurückgebliebenen nichtbrennbaren Aufschlämmung und Neutralwaschen mit Wasser isoliert werden. Man erhält ein echt gelbstichigrotes Produkt mit deutlich verbesserter Farbstärke im Vergleich zu einem handelsüblichen $\gamma_{II}$-Produkt ähnlicher Partikelgrösse. Das Pigment zeigt eine ausgezeichnete Wetterbeständigkeit und sehr gute Deckkraft. Dank diesen Eigenschaften ist es sehr geeignet für die Herstellung von Lacken, insbesondere Automobillacken, allein oder im Gemisch mit anderen verträglichen Pigmenten. Die erfindungsgemäss erhältliche $\gamma_I$-Form besitzt den ausgeprägtesten Gelbstich aller bekannten Formen von unsubstituiertem Chinacridon.

Das erfindungsgemäss erhältliche Pigment lässt sich beispielsweise als Pulver, Teig, Flushpaste und Zubereitung anwenden und eignet sich z.B. für Druckfarben, Leimfarben, Binderfarben oder Lacke aller Art, wie physikalisch und oxydativ trocknende Lacke, säure-, amin- und peroxidhärtende Lacke oder Polyurethanlacke. Das Pigment kann auch zum Färben von synthetischen, halbsynthetischen oder natürlichen makromolekularen Stoffen verwendet werden, wie Polyvinylchlorid, Polystyrol, Polyäthylen, Polyestern, Phenoplasten, Aminoplasten und Gummi. Weitere Anwendungsgebiete sind das Färben von natürlichen, regenerierten oder künstlichen Fasern, wie Glas-, Silikat-, Asbest-, Holz-, Cellulose-, Acetylcellulose-, Polyacrylnitril-, Polyester-, Polyurethan- und Polyvinylchloridfasern oder deren Gemischen, eventuell zusammen mit anderen organischen oder anorganischen Pigmenten. Man erhält mit dem neuen Pigment Drucke, Lackierungen, Anstriche, Beläge, Beschichtungen, geformte Gebilde, wie Folien, Fäden, Platten, Blöcke, Granulate und Stäbe, mit brillanter roter Farbe von hervorragender Dauerhaftigkeit.

Die erfindungsgemäss erhältlichen, farbstarken gelbstichigroten und deckenden Pigmente können zum Färben von festen, elastischen, pastenartigen, dickflüssigen, dünnflüssigen oder thixotropen Massen verwendet und in diese nach an sich bekannten Verfahren eingearbeitet werden. Wasserhaltige Teige können beispielsweise durch Einrühren des Pigments in Wasser, gegebenenfalls unter Zusatz eines Netz- oder Dispergiermittels oder durch Einrühren oder Einkneten des Pigments in ein Dispergiermittel in Gegenwart von Wasser und gegebenenfalls von organischen Lösungsmitteln oder Oelen erhalten werden. Diese Teige können beispielsweise wiederum zur Herstellung von Flushpasten, Druckfarben, Leimfarben, Kunststoffdispersionen und Spinnlösungen verwendet werden. Das Pigment kann aber auch durch Einrühren, Einwalzen, Einkneten oder Einmahlen in Wasser, organische Lösungsmittel, nicht trocknende Oele, trocknende Oele, Lacke, Kunststoffe oder Gummi gebracht werden. Schliesslich ist es auch möglich, das Pigment durch trockenes Mischen mit organischen oder anorganischen Massen, Granulaten, Faserstoffen, Pulvern und anderen Pigmenten zu Stoffmischungen zu verarbeiten.

Das erfindungsgemäss erhältliche Pigment zeichnet sich nicht nur durch ausgezeichnete Deckkraft, hervorragende Reinheit des Farbtons, und gute Allgemeinechtheiten, wie Licht- und Wetterbeständigkeit und Unempfindlichkeit gegen Lösungsmittel und Weichmacher, sondern auch durch seine vorzügliche Hitzebeständigkeit, aus. Dadurch kann das erfindungsgemäss erhältliche Pigment, im Gegensatz zu den bisher bekannten Chinacridon-Modifikation, in Polyethylen hoher oder niedriger Dichte oder in Polypropylen eingearbeitet werden, ohne dass die Farbe durch die hohen Verarbeitungstemperaturen abgestumpft wird. Die Ueberlegenheit des $\gamma_I$-Pigments bezüglich Deckkraft und Farbtonreinheit zeigt sich z.B. deutlich in Automobillackierungen. Um die gleiche Deckkraft zu erzielen, muss eine kleinere Menge an neuem Produkt eingesetzt werden, als es an bekanntem γ-Chinacridon nötig wäre. Die Lackierungen zeigen auch einen höheren Glanz.

In den nachfolgenden Beispielen, die die vorliegende Erfindung weiter erläutern, sind Teile, wenn nicht anders definiert, als Gewichtsteile und Prozente als Gewichtsprozente ausgedrückt.

Beispiel 1: In eine handelsübliche Mühle (2,84 l) werden 2500 Teile Keramikperlen von 1,6-2,5 mm Durchmesser, bestehend im Durchschnitt aus ca. 69 % $ZrO_2$ und 31 % $SiO_2$, 50 Teile rohes $\gamma_{II}$-Chinacridon und 5,0 Teile wasserfreies Natriumsulfat gegeben .Die Mühle wird bei Raumtemperatur 10 Stunden bei 68 U/Min., ca. 74 % der kritischen Geschwindigkeit, gedreht ("kritische Geschwindigkeit" ist die Geschwindigkeit, bei der die Fliehkraft die Schwerkraft aufhebt, so dass die Mahlhilfsmittel gegen die Wand der Mühle gehalten werden).

Ein kleiner Anteil (36 Teile) an mit Pigment beschichteten Perlen wird von der Mühle entfernt und das Pigment wird mit Methanol ausgewaschen. Die Aufschlämmung wird filtriert und das trockene Produkt einer Röntgenbeugungsanalyse unterworfen. Das Produkt zeigt geringere Kristallinität als das Ausgangsprodukt und besteht im wesentlichen aus $\gamma_I$-Chinacridon.

In die Kugelmühle werden dann 791 Teile Methanol und anschliessend 75 Teile 43,5 %ige wässrige Kalilauge gegeben und die Mühle 48 Stunden mit gleicher Geschwindigkeit, wie für die erste Stufe gedreht. Die Mühle wird in einem Sieb, das die Perlen zurückhält, gelehrt und die Perlen mit etwa 1000 Teilen Wasser gewaschen, bis das Pigment praktisch vollständig in der abfiltrierten Aufschlämmung aufgefangen wird. Die basische Aufschlämmung wird in einem Destillationskolben übergeführt und das Methanol wird, zusammen mit etwas Wasser, abdestilliert. Die Temperatur steigt während der Destillation langsam an. Wenn sie 90°-93°C erreicht hat, wird die Destillation abgebrochen. Das Produkt wird duch Filtrieren abgetrennt und das Filtergut mit Wasser neutral gewaschen. Nach dem Trocknen bei 80°C erhält man 47,6 Teile Pigment.

Nach üblichen Methoden in einem Oeldruckfarben-System eingearbeitet (z.B. durch Ausreiben auf einem Hoover-Muller in lithographischem Firnis), ergibt das Pigment Ausfärbungen, die sich insbesondere durch den Gelbstich und durch die hohe Farbstärke sowohl im Vollton, wie auch Weissverschnitt ($TiO_2$) auszeichnen.

Beispiel 2: In eine handelsübliche Mühle (2,84 l) werden 2500 Teile Keramikperlen von 1,6-2,5 mm Durchmesser, bestehend im Durchschnitt aus ca. 69 % $ZrO_2$ und 31 % $SiO_2$, 50 Teile rohes $\gamma_{II}$-Chinacridon, 791 Teile Methanol und 75 Teile 43,5 %ige wässrige Kalilauge gegeben. Die Mühle wird bei Raumtemperatur 72 Stunden bei 68 U/Min., etwa 74 % der kritischen Geschwindigkeit, gedreht. Die Mühle wird in einem Sieb, das die Perlen zurückhält, gelehrt und die Perlen mit etwa 1000 Teilen Wasser gewaschen, bis das Pigment praktisch vollständig in der abfiltrierten Aufschlämmung aufgefangen wird. Die basische Aufschlämmung wird in einem Destillationskolben übergeführt und das Methanol wird, zusammen mit etwas Wasser, abdestilliert. Die Temperatur steigt während der Destillation langsam an. Wenn sie 90°-93°C erreicht hat, wird die Destillation abgebrochen. Das Produkt wird durch Filtrieren abgetrennt und das Filtergut mit Wasser neutral gewaschen. Nach dem Trocknen bei 80°C erhält man 48,2 Teile Pigment.

In einem Oeldruckbfarben-System eingearbeitet, ergibt das Pigment Ausfärbungen, die mit derjenigen von Beispiel 1 im wesentlichen identisch sind.

Beispiel 3:

a) Eine Laborkugelmühle wird mit 871 Teilen Keramikstäben (12,7 ∅), 50 Teilen rohem $\gamma_{II}$-Chinacridon und 5,0 Teilen wasserfreiem Natriumsulfat beschickt. Sie wird dann bei Raumtemperatur 24 Stunden bei etwa 75 % der kritischen Geschwindigkeit gedreht und anschliessend in ein Sieb, das die Stäbe zurückhält gelehrt.

b) Ein Anteil des Mahlpulvers von a) (56 Teile) wird in eine Labormühle (2,84 l) mit 2500 Teilen Keramikperlen von 1,6-2,5 mm Durchmesser, 791 Teilen Methanol und 75 Teilen 43,5 %ige wässrige Kalilauge gegeben. Vermahlung und Aufarbeitung werden, wie in Beispiel 1 beschrieben, durchgeführt. Das erhaltene Produkt ist bezüglich Röntgenbeugungsdiagramm und Eigenschaften in Oeldruckfarben-System identisch mit demjenigen von Beispiel 1.

c) Ein weiterer Anteil des Mahlpulvers von a) (11 Teile) wird in 158,2 Teilen Methanol, das 15 Teile 43,5 %ige Kalilauge enthält, dispergiert, kräftig gerührt und 48 Stunden am Rückfluss gekocht. Das Pigment wird nach Zusatz von Wasser und Abdestillieren des Methanols isoliert. Das Produkt besteht im wesentlichen aus $\gamma_I$-Chinacridon, ist aber bezüglich der Farbe etwas abweichend vom gemäss b) vermahlenen Produkt, nämlich etwas dunkler und stumpfer im Vollton und etwas farbschwächer im Weissverschnitt. Obwohl durch das Rückfluss-Verfahren auch $\gamma_I$-Chinacridon hergestellt werden kann, wird das Mahlverfahren bevorzugt, da die Pigmenteigenschaften, insbesondere bei Volltonausfärbungen, überlegen sind.

Beispiel 4: 6 Teile rohes $\gamma_{II}$-Chinacridon und 0,6 Teile wasserfreies Natriumsulfat werden in eine Laborkugelmühle enthaltend 300 Teile Keramikperlen von 1,6-2,5 mm Durchmesser gegeben. Die Mühle wird dann bei Zimmertemperatur 7 1/2 Stunden bei etwa 75% der kritischen Temperatur gedreht. Danach wird die Mühle geöffnet, 79 Teile Methanol und 10 Teile 50 %iger wässriger Natronlauge werden zugegeben und die Mühle wird anschliessend noch 72 Stunden gedreht. Die erhaltene Aufschlämmung wird wie in Beispiel 1 beschrieben aufgearbeitet. Man erhält 5,3 Teile Pigment. Das Produkt besteht im wesentlichen aus $\gamma_I$-Chinacridon

und zeigt in der Applikation die gleichen Eigenschaften, wie ein entsprechendes aber in Gegenwart von Kalilauge hergestelltes Produkt, mit der einzigen Ausnahme, dass es leicht farbschwächer ist.

Beispiel 5: Beispiel 1 wird wiederholt mit der Ausnahme, dass die Nassmahlstufe bei 49-54°C erfolgt. Man erhält ein Produkt hoher Kristallinität, das im wesentlichen aus $\gamma_I$-Chinacridon besteht. Die damit erhaltenen Ausfärbungen sind gelbstichiger im Vollton, deckender und leicht farbschwächer im Weissverschnitt als diejenigen mit dem Pigment aus Beispiel 1.

Beispiel 6: Eine Laborkugelmühle wird mit 870 Teilen Keramikkugeln von 12,7 mm Durchmesser mit hohem Aluminiumoxidgehalt, 50 Teilen rohem $\gamma_{II}$-Chinacridon und 5,0 Teilen wasserfreiem Natriumsulfat beschickt. Sie wird dann bei Raumtemperatur 24 Stunden bei etwa 75 % der kritischen Geschwindigkeit gedreht und anschliessend in ein Sieb, das die Kugeln zurückhält, gelehrt. Die Kugeln werden, um die Ausbeute an Vorvermahlprodukt zu erhöhen, gerührt.

Ein Anteil am erhaltenen Mahlpulver (82 Teile) wird in eine Labormühle (2,84 l) enthaltend 2500 Teile Keramikperlen von 1,6-2,5 mm Durchmesser, 791 Teile Methanol und 75 Teile 43,5 %ige Kalilauge gegeben. Dann wird die Mühle wieder wie oben, aber während 72 Stunden gedreht. Die Aufarbeitung erfolgt wie im Beispiel 1 beschrieben. Man erhält ein Produkt, das im wesentlichen aus $\gamma_I$-Chinacridon besteht und das in der Applikation die gleichen Eigenschaften besitzt, wie dasjenige von Beispiel 1.

Wenn man die Vorvermahlung auf 48 Stunden verlängert, kann der Ansatz an Mahlpulver in der zweiten Stufe von 82 auf 110 Teile erhöht werden.

Beispiel 7: In eine handelsübliche Mühle (2,84 l) werden 2500 Teile Keramikperlen, $\gamma_{II}$-Chinacridon und Natriumsulfat, wie in Beispiel 1 beschrieben, vermahlen. Die Mühle wird gelehrt. Die Keramikperlen sind mit vorvermahlenem Pigment beschichtet. 300 Teile dieses Materials, 81 Teile n-Butanol und 7,5 Teile 43,5 %ige wässrige Kalilauge werden in eine Laborkugelmühle gegeben. Diese wird dann bei Raumtemperatur 72 Stunden bei etwa 75 % der kritischen Geschwindigkeit gedreht. Die Pigmentaufschlämmung wird von den Keramikperlen abgetrennt und letztere mit 150 Teilen Wasser gewaschen. Die vereinigten Aufschlämmungen werden geheizt und der n-Butylalkohol abdestilliert, bis die Temperatur der Aufschlämmung 100°C erreicht hat. Das Pigment wird abafiltriert und mit Wasser neutral gewaschen. Man erhält 4,9 Teile Chinacridon der $\gamma_I$-Modifikation von kleinerer Partikelgrösse als dasjenige von Beispiel 1. In einem Oeldruckfarben-System eingearbeitet, ergibt es Ausfärbungen, die etwas stumpfer und transparenter im Vollton und etwas farbstärker im Weissverschnitt ($TiO_2$), im Vergleich zum Produkt von Beispiel 1, sind.

Beispiel 8: Das Produkt von Beispiel 1 (91 %) wird zusammen mit 3 % 2-Phthalimidomethylchinacridon und 6 % eines polymerischen Dispergiermittels (®Disperbyk 160, BYK-Chemie) gepulvert. In ein Alkydlack-System eingearbeitet, zeigt das Produkt ein hervorragendes rheologisches Verhalten und ergibt Lackierungen von erstaunlich hohem Glanz.

**Patentansprüche**

1. Verfahren zur Umwandlung von rohem Chinacridon der $\gamma_{II}$-Modifikation in Chinacridon der $\gamma_I$-Modifikation mit einer durchschnittlichen mittleren Partikelgrösse von mehr als 0,1 µm und einer spezifischen Oberfläche von weniger als 30 $m^2/g$, dadurch gekennzeichnet, dass man das rohe $\gamma_{II}$-Chinacridon in Gegenwart einer zur Umwandlung genügenden Menge eines Alkohols und einer Base mahlt und anschliessend das gelbstichigrote $\gamma_I$-Chinacridon isoliert.

2. Verfahren gemäss Anspruch 1, dadurch gekennzeichnet, dass der Alkohol ein niedrigsiedender Alkanol oder ein Glykol ist.

3. Verfahren gemäss Anspruch 1, dadurch gekennzeichnet, dass der Alkohol Methanol, Ethanol, n-Butanol, n-Pentanol oder Ethylenglykol ist.

4. Verfahren gemäss Anspruch 1, dadurch gekennzeichnet, dass der Alkohol Methanol ist.

5. Verfahren gemäss Anspruch 1, dadurch gekennzeichnet, dass die Base ein Alkalimetallhydroxid ist.

6. Verfahren gemäss Anspruch 1, dadurch gekennzeichnet, dass die Base Kaliumhydroxid ist.

7. Verfahren gemäss Anspruch 1, dadurch gekennzeichnet, dass der Alkohol in einer 6- bis 18-fachen Menge, bezogen auf das Gewicht des Pigments, und die Base in einer Menge von 1,0 bis 10,0 Gew-% bezogen auf den Alkohol, eingesetzt werden.

8. Verfahren zur Umwandlung von rohem Chinacridon der $\gamma_I$-Modifikation in Chinacridon der $\gamma_I$-Modifikation mit einer durchschnittlichen mittleren Partikelgrösse von mehr als 0,1 µm und einer spezifischen Oberfläche von weniger als 30 $m^2/g$, dadurch gekennzeichnet, dass man

a) das rohe $\gamma_{II}$-Chinacridon einer Vorvermahlung zur Umwandlung in die rohe $\gamma_I$-Modifikation unterwirft und

b) das vermahlene Produkt mit einer zur Wachstumsförderung der Parikel genügenden Menge eines Alkohols und einer Base behandelt und anschliessend das gelbstichigrote $\gamma_I$-Chinacridon isoliert.

9. Verfahren gemäss Anspruch 8, dadurch gekennzeichnet, dass das vorvermahlene Produkt mit einer zur Wachstumsförderung der Partikel genügenden Menge eines Alkohols und einer Base am Rückfluss gekocht wird.

10. Verfahren gemäss Anspruch 8, dadurch gekennzeichnet, dass das vorvermahlene Produkt mit einer zur Wachstumsförderung der Partikel genügenden Menge eines Alkohols und einer Base vermahlen wird.

11. Verfahren gemäss Anspruch 8, dadurch gekennzeichnet, dass der Alkohol ein niedrigsiedender Alkanol oder ein Glykol ist.

12. Verfahren gemäss Anspruch 8, dadurch gekennzeichnet, dass der Alkohol Methanol, Ethanol, n-Butanol, n-Pentanol oder Ethylenglykol ist.

13. Verfahren gemäss Anspruch 8, dadurch gekennzeichnet, dass der Alkohol Methanol ist,

14. Verfahren gemäss Anspruch 8, dadurch gekennzeichnet, dass die Base ein Alkalimetallhydroxid ist.

15. Verfahren gemäss Anspruch 8, dadurch gekennzeichnet, dass die Base Kaliumhydroxid ist.

16. Verfahren gemäss Anspruch 8, dadurch gekennzeichnet, dass der Alkohol in einer 6- bis 18-fachen Menge, bezogen auf das Gewicht des Pigments, und die Base in einer Menge von 1,0 bis 10,0 Gew-%, bezogen auf den Alkohol, eingesetzt werden.